# FASCICULE DE BREVET EUROPEEN

(11) **EP 0 935 458 B1**
(45) Date de publication et mention de la délivrance du brevet: **10.04.2002**
(21) Numéro de dépôt: 97933736.7
(22) Date de dépôt: 15.07.1997
(51) Int. Cl.: A61K 9/127, A61K 48/00, C12N 15/88

(54) **COMPOSITIONS CONTENANT AU MOINS UN ACIDE NUCLEIQUE**
MINDESTENS EINE NUKLEINSÄURE ENTHALTENDE ZUSAMMENSETZUNGEN
COMPOSITIONS CONTAINING AT LEAST ONE NUCLEIC ACID

(30) Priorité: 16.07.1996 FR 9608844
(43) Date de publication de la demande: 18.08.1999
(73) Titulaire: CAPSULIS, 33600 Pessac (FR)
(72) Inventeur: MAHY, Patrick, F-33400 Talence (FR); ROUX, Didier, F-33700 Mérignac (FR); LAVERSANNE, René, F-33600 Pessac (FR); AMEDEE, Joelle, F-33600 Pessac (FR); FREUND, Olivier, F-33400 Talence (FR)
(74) Mandataire: Giraud, Françoise
(86) Numéro de dépôt international: FR9701304
(87) Numéro de publication internationale: WO9802144

(56) Documents cités:
- EP-A- 0 424 688
- WO-A-95/16437
- WO-A-95/18601
- WO-A-97/04748
- WO-A-97/10851
- DE-A- 4 005 152
- US-A- 4 394 448
- S. AKHTAR ET AL.: "interactions of antisense DNA oligonucleotide analogs with phospholipid membranes (liposomes)" NUCLEIC ACIDS RESEARCH, vol. 19, no. 20, 25 octobre 1991, EYNSHAM, OXFORD (GB), pages 5551-5559, XP002027215

## Description

L'invention concerne de nouvelles compositions contenant au moins un acide nucléique et leurs applications dans le domaine biomédical, en particulier en thérapie génique.

Jusqu'à la fin des années quatre-vingt, l'utilisation des liposomes en vue d'applications biomédicales et, plus précisément, en thérapie génique ne semblait guère prometteuse. Les résultats de transfection (traduction et expression d'un gène de cellules procaryotes ou eucaryotes par des cellules eucaryotes), essentiellement transitoire, étaient relativement médiocres. Le recours aux enveloppes externes de virus a accru très significativement les résultats des transferts de gènes dans une multitude de lignées cellulaires *in vitro.* Par contre, il est certain que leur utilisation en thérapie humaine et animale soulève des problèmes étant donné les dangers iatrogènes possibles qu'elles représentent. Vers la fin de la décennie précédente, il est apparu que l'utilisation de tensioactifs cationiques améliorait très nettement les résultats de transfection. Depuis, malgré certains problèmes liés à l'utilisation des vecteurs cationiques de nombreuses formulations ont été proposées dont certaines sont brevetées et commercialisées.

Trois sortes de vecteurs non-viraux sont en général utilisés :
- des polymères cationiques,
- des vecteurs biochimiques constitués d'une protéine cationique associée à un récepteur cellulaire,
- des lipides cationiques associés ou non à des liposomes.

Tous ces vecteurs contiennent des molécules cationiques.

L'utilisation de tels vecteurs se trouve notamment décrite dans les publications suivantes :
J.P. Behr et al. Proc. Nat. Ac. Scienc. 86, 6982, 1989
M. Cotten et Wagner Curr. Opin. Cell Biol. 4, 705, 1993
J. Haensler and F.C. Szoka Bioconjug. Chem. 4, 372, 1993
C.P. Hodgson Bio Tcchnology 13, 222, 1995
F.D. Ledley Hum. Gene Ther. 6, 1129, 1995
J.S. Remy et al. Proc. Nat. Ac. Scienc. 92, 1744, 1995
V.S. Trubettskoy et al. Biochem. Biophys, Acta 1131, 311, 1992

Deux problèmes essentiels relèvent de l'utilisation des vecteurs cationiques :
1 - ces tensioactifs sont généralement cytotoxiques et même si certaines molécules spécifiques ont été développées afin d'en réduire la toxicité, ce problème n'est pas entièrement résolu,
2 - on ne peut pas envisager leur application *in vivo* car ces vecteurs, du fait de leur charge, interagissent très fortement avec les protéines, par exemple, avec celles présentes dans le sérum, ainsi qu'avec les parois cellulaires. Ils se collent donc rapidement sur les cellules voisines du site d'injection, en réduisant la diffusion systémique.

C'est pourquoi ils trouvent leur utilisation préférentielle *in vitro* pour des cellules en culture, dans le transfert de gènes.

Il est bien connu que les liposomes sont des structures colloïdales comportant un coeur aqueux séparé du milieu extérieur par une ou plusieurs bicouches de molécules phospholipidiques. Leur application en cosmétique et en pharmacologie a fait l'objet d'un grand nombre de brevets décrivant de multiples utilisations de ces vecteurs depuis leur découverte dans les années 1960.

Du point de vue des applications, on a très vite distingué les liposomes composés d'une seule bicouche, des liposomes composés de plusieurs bicouches souvent désignés par l'abréviation MLV correspondant à la terminologie anglaise "Multi-Layered Vesicles". -Les vésicules multicouches ont une taille qui est mal contrôlée et est généralement largement supérieure au micromètre. Parmi les vésicules unilamellaires, on distingue les petites vésicules souvent désignées par l'abréviation SUV correspondant à la terminologie anglaise "Small Unilamellar Vesicles" dont la taille ne dépasse pas 100 à 300 nm et les grandes vésicules souvent désignées par l'abréviation LUV correspondant à la terminologie anglaise "Large Unilamellar Vesicles" qui peuvent atteindre plusieurs dizaines de micromètres de taille.

Du fait de leur taille importante, les vésicules multilamellaires (MLV) n'ont que peu d'utilité dans les applications médicales. En effet, les risques d'embolie que comporte l'introduction de particules de taille supérieure au micromètre dans la circulation sanguine rendent leur utilisation impossible par injection intra-veineuse. De plus, une taille trop importante empêche les vésicules de franchir les barrières tissulaires, permettant de les retrouver dans le sang lorsqu'elles sont injectées par voie intra-musculaire ou sous-cutanée. Enfin, il n'existe pas de procédés réellement efficaces pour produire des MLV de façon parfaitement contrôlée. La plus grande partie des applications ont été développées avec des SUV qui répondent aux critères de sécurité et de contrôle nécessaires à une utilisation biomédicale.

On a également décrit dans la littérature l'encapsulation d'acides nucléiques dans des vésicules à base de tensioactifs, généralement lipidiques. Toutes les vésicules décrites à ce jour présentent en commun le fait de présenter plusieurs couches de tensioactifs entourant un coeur liquide. De telles vésicules sont décrites, en particulier, dans les brevets US 4,394,448 et WO-95/16437.

Il a été décrit dans l'article extrait de "Nucleic Acids Research", vol. 19, n° 20, 5551-5559, des expériences de diffusion d'oligonucléotides dans des membranes phospholipidiques. Ce document est une étude sur l'interaction entre membranes phospholipidiques et oligo-nucléotides dans laquelle les liposomes, qui présentent un coeur aqueux central, sont utilisés pour modéliser ces membranes.

L'un des problèmes pratiques de l'utilisation des liposomes pour la vectorisation des médicaments ou leur utilisation en thérapie génique est le faible rendement dû au fait que le pourcentage de la solution aqueuse de départ effectivement encapsulée dépasse rarement 30 %. De plus, la méthode généralement suivie fait appel à des étapes d'évaporation de solvants organiques intervenant dans le procédé de fabrication. Enfin, les résultats expérimentaux se sont révélés très décevants.

En effet, le processus communément admis pour l'incorporation dans les cellules des vésicules lipidiques, ou des complexes cationiques contenant de l'ADN, passe par un mécanisme d'endocytose. Dans le cytoplasme, l'objet en cours de pénétration est inclus dans un endosome qui contient des enzymes, en particulier des DNases, capables de détruire tout matériel génétique intrus. Les liposomes, du fait de leur faible nombre de membranes lipidiques entourant le coeur aqueux ne présentent pas une protection suffisante pour résister à l'action destructrice des protéases et nucléases endosomiales. De ce fait, même s'ils sont efficaces pour pénétrer le cytoplasme de la cellule, les liposomes ne permettent à l'ADN d'atteindre le noyau qu'avec un très faible rendement.

Il est donc crucial de développer des vecteurs synthétiques compatibles avec une utilisation *in vivo* et, ceci, d'autant plus qu'il n'existe pas, à l'heure actuelle, un vecteur efficace pour des molécules susceptibles d'être appliquées en thérapie génique humaine et/ou animale. Seules, les enveloppes virales montrent une efficacité en transfection compatible avec une application humaine ou animale.

Les vésicules de type liposomes ou vésicules paucilamellaires ont en commun d'être constituées d'une ou plusieurs couches lamellaires entourant un coeur aqueux. Outre ce type de vésicules, on connaît également des vésicules multilamellaires qui se différencient structurellement des précédentes par le fait qu'elles présentent une structure dite en "oignon" et sont constituées, de leur centre jusqu'à leur périphérie, d'une succession de couches lamellaires séparées par un milieu liquide. Ces vésicules peuvent être obtenues par un procédé comprenant la préparation d'une phase lamellaire cristal-liquide et sa transformation par application d'un cisaillement. Un tel procédé est en particulier décrit dans le brevet WO 93/19735 issu du brevet français FR-2 689 418 ou WO 95/18601.

Selon le brevet français FR-2 689 418, cette transformation peut être faite lors d'une étape de cisaillement homogène de la phase cristal-liquide, ce qui conduit à des vésicules encore appelées microcapsules de taille contrôlée. Toutefois, en jouant sur la formulation de la phase lamellaire cristal-liquide, en particulier sur la nature des tensioactifs entrant dans sa composition, la transformation de cette phase cristal-liquide en vésicules peut être obtenue par simple sollicitation mécanique, en particulier lors du mélange des constituants.

Les recherches menées par les inventeurs les ont amenés à découvrir que l'utilisation des technologies décrites ci-dessus permettait la mise au point de nouveaux vecteurs multilamellaires de petite taille, non cationiques, permettant d'encapsuler, de protéger et de délivrer de l'ADN dans des cellules et, ceci, avec une grande efficacité d'encapsulation ainsi qu'une grande facilité de préparation.

Un autre avantage est que toutes les molécules entrant dans la préparation des compositions de l'invention sont disponibles commercialement.

Un autre avantage du procédé permettant la préparation des compositions de l'invention est qu'il permet d'utiliser une grande variété de tensioactifs.

Selon un autre avantage, l'invention fournit un véhicule permettant, vraisemblablement du fait de sa structure spécifique, de protéger l'acide nucléique des agressions extérieures, en particulier des agressions enzymatiques. Ce point est illustré par l'exemple 1. Ceci constitue un net avantage par rapport aux liposomes et aux vésicules paucilamellaires classiques qui ne sont que très peu efficaces en transfection, probablement parce qu'ils ne sont pas capables de protéger l'ADN de l'action des DNases présentes en particulier dans l'endosome. La force de la technologie de l'invention est en partie due à cette protection des acides nucléiques vis-à-vis des nucléases. On peut supposer, sans que cela constitue une explication certaine dans l'état actuel des connaissances des inventeurs, que les premières couches de la vésicule multilamellaire sont effectivement détruites par l'action enzymatique dans l'endosome, mais qu'il reste suffisamment d'ADN encapsulé lors de la rupture de l'endosome, pour avoir libération de cet ADN dans le cytoplasme, ainsi capable d'atteindre le noyau. De plus, la forte concentration en tensioactifs dans les vésicules de l'invention pourrait favoriser, lors de l'érosion des premières couches, la déstabilisation de l'endosome par action des tensioactifs libérés sur les parois de l'endosome.

Selon un autre de ses avantages, l'invention permet, le cas échéant, d'incorporer dans les compositions, des produits à caractère cationique déjà connus pour leur intérêt en transfection, les autres ingrédients de la composition de l'invention permettant de masquer le caractère cationique de cette molécule, permettant ainsi de l'utiliser pour des applications *in vivo*. Ainsi, l'invention fournit donc, en outre, un moyen d'augmenter l'efficacité ou de diminuer la toxicité d'une molécule cationique connue pour son utilisation en transfection. En effet, la plupart des composés cationiques utilisés sont cytotoxiques et on peut diminuer cette cytotoxicité en encapsulant ces molécules, ce qui constitue un avantage complémentaire de l'invention.

D'autres avantages découlent de la description et des exemples qui suivent d'où il ressort que ce type de vecteur permet de surmonter sensiblement tous les problèmes exposés précédemment et d'offrir, pour la première fois, un véhicule sous forme de vésicules à base de tensioactifs utilisable *in vivo*.

L'invention concerne, selon l'une de ses caractéristiques essentielles, une composition contenant au moins un acide nucléique dont au moins une partie se trouve incluse à l'intérieur de vésicules multilamellaires, caractérisée en ce que lesdites vésicules sont constituées de leur centre jusqu'à leur périphérie de bicouches comprenant au moins un agent tensioactif, concentriques et séparées par un milieu liquide. Des vésicules présentant une telle structure seront ci-après désignées par vésicules à "structure en oignon".

Par structure en "oignon", on entend, comme exposé précédemment, une structure multilamellaire, dans laquelle les vésicules de forme sensiblement sphérique sont constituées d'une succession de bicouches concentriques et, cela, du centre à la périphérie des vésicules, d'où le nom de structure en oignon utilisé, par analogie, pour qualifier de telles structures.

De telles structures sont avantageusement obtenues par incorporation d'au moins un acide nucléique dans une phase lamellaire cristal-liquide comprenant au moins un agent tensioactif puis transformation de cette phase cristal-liquide lamellaire en une phase dense de vésicules multilamellaires de petite taille.

Ainsi, selon une autre caractéristique essentielle de l'invention, elle concerne un procédé de préparation d'une composition contenant au moins un acide nucléique telle que définie précédemment, selon lequel on prépare une phase cristal-liquide lamellaire incorporant ledit acide nucléique et on provoque le réarrangement de ladite phase cristal-liquide en vésicules multilamellaires par application d'un cisaillement.

Ce cisaillement pourra être un cisaillement homogène, ce qui présente l'avantage de conduire à des vésicules de taille parfaitement homogène. Toutefois, une simple agitation mécanique pourra s'avérer suffisante pour conduire à la formation des vésicules multilamellaires de l'invention.

Selon une autre caractéristique encore, l'invention concerne les produits susceptibles d'être obtenus par ce procédé.

Selon le brevet français FR-2 689 418, cette transformation peut être faite lors d'une étape de cisaillement homogène de la phase cristal-liquide, ce qui conduit à des vésicules ou microcapsules de taille contrôlée. Toutefois, en jouant sur la formulation de la phase lamellaire cristal-liquide, en particulier sur la nature des tensioactifs entrant dans sa composition, la transformation de cette phase cristal- liquide en vésicules peut être obtenue par simple sollicitation mécanique, en particulier lors du mélange des constituants.

La formulation fait avantageusement intervenir un mélangé de molécules tensioactives. Il est généralement utilisé au moins deux tensioactifs différents ayant des balances hydrophile lipophile différentes, ce qui permet de régler en continu les propriétés des bicouches et ainsi de contrôler l'apparition de l'instabilité qui gouverne la formation des vésicules multilamellaires.

Selon une variante avantageuse, les vésicules constituant les compositions de la présente invention ont des dimensions inférieures à 1 µm, de préférence comprises entre 0,1 et 1 µm.

Selon une autre variante avantageuse de l'invention, les tensioactifs constituant les bicouches des vésicules sont des tensioactifs non cationiques, ce qui présente, comme on l'a vu précédemment, un gros avantage par rapport aux compositions de l'art antérieur mettant en oeuvre des molécules cationiques.

Selon une variante avantageuse, les membranes des vésicules contenues dans les compositions de l'invention contiennent avantageusement au moins un agent tensioactif choisi dans le groupe constitué :
- des phospholipides hydrogénés ou non hydrogénés,
- des acides gras en C₆ à C₁₈, saturés ou mono- ou polyinsaturés, linéaires ou ramifiés, sous forme d'acide ou de sel d'un métal alcalin, alcalino-terreux ou d'une amine,
- des esters, éthoxylés ou non, de ces mêmes acides gras et
   . de saccharose,
   . de sorbitan
   . de mannitol,
   . de glycérol ou de polyglycérol,
   . de glycol,
- des mono-, di- ou triglycérides ou des mélanges de glycérides de ces mêmes acides gras,
- des alcools gras en C₆ à C₁₈, saturés ou mono- ou polyinsaturés, linéaires ou ramifiés, éthoxylés ou non,
- des éthers, éthoxylés ou non, de ces mêmes alcools gras et
   . de saccharose,
   . de sorbitan,
   . de mannitol,
   . de glcérol ou de polyglycérol,
   . de glycol,
- des huiles végétales polyéthoxylées, hydrogénées ou non hydrogénées,
- des polymères séquencés de polyoxyéthylène et de polyoxypropylène (poloxamères),
- de l'hydroxystéarate de polyéthylèneglycol,
- des alcools à squelette stérol tel que le cholestérol, le sistostérol.

Selon une variante avantageuse, les tensioactifs entrant dans la composition des bicouches des vésicules sont constitués de tensioactifs non cationiques.

Les tensioactifs choisis, en particulier les tensioactifs cités ci-dessus, sont avantageusement choisis dans la catégorie des tensioactifs permis par la législation pour l'utilisation pharmaceutique en fonction de la voie d'administration.

On choisira avantageusement parmi les tensioactifs ci-dessus, deux tensioactifs présentant des propriétés relativement différentes, en particulier une balance hydrophile lipophile (HLB) différente. Le premier tensioactif présentera avantageusement une balance hydrophile lipophile comprise entre 1 et 6 alors que le deuxième tensioactif aura une balance hydrophile lipophile comprise entre 3 et 15.

Comme on l'a vu précédemment, un meilleur contrôle de la taille des vésicules multicouches pourra être obtenu en procédant selon le brevet FR 2 689 418.

La préparation obtenue après transformation de la phase lamellaire cristal-liquide en vésicules multilamellaires peut ensuite être diluée, en particulier avec un solvant aqueux pour obtenir ainsi une suspension aqueuse de vésicules.

Comme exposé précédemment, un des inconvénients des vecteurs d'acides nucléiques connus à ce jour, en particulier des vecteurs de type liposome, est de ne permettre que rarement de dépasser des rendements d'encapsulation de 30 %. Le procédé utilisé pour préparer les vésicules selon l'invention permet, quant à lui, d'atteindre des rendements d'encapsulation qui peuvent être voisins de 100 %. Toutefois, un tel rendement d'encapsulation, du fait de la grande activité obtenue grâce à la structure particulière des vésicules de l'invention ne s'avère pas toujours nécessaire.

Ainsi, le rendement d'encapsulation du(des) acide(s) nucléique(s) dans les compositions de l'invention est avantageusement d'au moins 10 %, de préférence d'au moins 40 % mais peut également être compris entre 60 et 100 %, ce qui représente un avantage supplémentaire par rapport aux technologies de l'art antérieur.

Les compositions de l'invention contenant des vésicules renfermant au moins un acide nucléique peuvent être utilisées pour des applications *in vitro*, en particulier pour transformer une lignée cellulaire, en particulier pour l'immortaliser ou en modifier l'expression d'un ou plusieurs gènes.

L'invention fournit un moyen de vectorisation d'acides nucléiques, cette vectorisation pouvant être réalisée aussi bien *in vivo* que *in vitro.*

Ces vésicules peuvent également être utilisées pour la préparation de compositions pharmaceutiques utilisables en thérapie génique.

A titre d'exemples d'acide nucléique que peuvent renfermer les vésicules de l'invention, on citera l'ADN ou une séquence nucléotidique d'ADN.

Les vésicules peuvent également renfermer un gène particulier ou une séquence de ce gène, plus particulièrement une séquence codant pour une protéine donnée.

Les compositions de l'invention peuvent également inclure dans les vésicules de l'ARN ou une séquence nucléotidique d'ARN.

On peut également, selon une autre variante de réalisation, inclure dans les vésicules un oligonucléotide, cet oligonucléotide pouvant être de type sens et/ou anti-sens.

Les vésicules de l'invention ont avantageusement une taille inférieure à 1 µm, de préférence comprise entre 0,1 et 1 µm. Cette taille pourra être contrôlée et limitée par l'application d'un cisaillement homogène sur la phase cristal-liquide selon le procédé du brevet FR-2 689 418 ou WO 9319735.

Selon des variantes particulièrement intéressantes de l'invention, différents produits pourront être co-encapsulés avec l'acide nucléique en vue, en particulier, soit d'améliorer les conditions d'action de cet acide nucléique, soit d'apporter des fonctionnalités complémentaires à celles du produit encapsulé.

De telles compositions pourront être produites par le procédé précédemment décrit, en incorporant le produit à co-encapsuler en combinaison avec l'acide nucléique dans la phase cristal-liquide.

Parmi les produits que l'on co-encapsulera avantageusement avec les acides nucléiques, on citera, en particulier, les produits de type "agents de condensation" qui forment avec l'acide nucléique une sorte de complexe favorisant le "repliement" de la chaîne d'acide nucléique, ce qui favorise sa stabilité en présence de nucléases.

A titre d'exemple préféré d'agents de condensation, on citera les histones qui sont des protéines, existant naturellement dans le noyau pour condenser l'ADN. Les histones présentent la particularité de rester étroitement liées à l'ADN et sont ainsi susceptibles de jouer un rôle important dans beaucoup de réactions génomiques. Parmi les histones que l'on encapsulera avantageusement en combinaison avec au moins un acide nucléique, en particulier avec l'ADN, on citera les histones dites nucléosomiques, responsables du repliement de l'ADN en nucléosome, plus précisément les histones H2A, H2B, H3 et H4 et l'histone H1 qui ne participe pas directement au repliement de l'ADN en nucléosome , mais à l'empilement de ces derniers. L'utilisation d'un mélange d'histones H1, H2A, H2B H3 et H4 (issues du thymus de veau) utilisées avec un rapport de 1mg de protéines totales/mg d'ADN a ainsi permis de condenser 50 mg d'ADN avec un rendement d'encapsulation maximal dans les vésicules de l'invention.

Selon d'autres variantes de l'invention, on co-encapsulera avantageusement dans la vésicule multilamellaire de l'invention différentes enzymes, en particulier des enzymes d'intégration, de recombinaison ou des enzymes destinées à optimiser la réplication de l'acide nucléique encapsulé telles que les topoisomérases ou les hélicases.

Ainsi, selon une variante de l'invention, on pourra co-encapsuler dans la vésicule multilamellaire des enzymes d'intégration dont la fonction est de permettre au gène introduit dans une cellule de s'exprimer dans la cellule hôte, mais surtout de s'intégrer au génome, pour donner des lignées de cellules filles gardant la fonction apportée par le gène introduit. A ce jour, seule l'utilisation des rétrovirus comme vecteur d'ADN permet un transfert de gènes stable et efficace, de par l'intégration du génome viral dans la cellule hôte. Toutefois, on ne peut écarter tous les problèmes liés à l'utilisation des rétrovirus dans le domaine biomédical. Ainsi, la force de la technologie de l'invention est de pouvoir co-encapsuler l'ADN avec des protéines, en particulier des enzymes permettant cette intégration appelées les enzymes d'intégration. On peut ainsi apporter dans un même vecteur l'ADN et les outils nécessaires à son incorporation.

Il est ainsi possible de stimuler cette intégration génomique par l'addition d'enzymes de recombinaison spécifiques du site qui permettent d'introduire, voire d'éliminer, des séquences nucléotidiques données. Cette forme de recombinaison est appelée recombinaison spécifique de site car l'enzyme de recombinaison appelée intégrase reconnaît des séquences nucléotidiques pouvant être introduites dans un vecteur d'ADN susceptible de se recombiner avec l'ADN de la cellule hôte. Cette enzyme de recombinaison rapproche les sites spécifiques, initie la réaction de coupure et de soudure de l'ADN.

D'autres outils enzymatiques peuvent également être intégrés dans le vecteur. L'ADN est répliqué chez les organismes eucaryotes en forme de chromatine dans laquelle l'ADN est fortement associé aux histones, protéines qui peuvent être également utilisées selon la présente invention pour optimiser la quantité d'ADN encapsulé. Si cette structure condensée en nucléosomes empilés, agit comme une barrière qui stopperait par exemple, les enzymes responsables de la réplication, l'association de l'ADN condensé avec des enzymes telles que les topoisomérases ou les hélicases permet de résoudre les éventuels problèmes d'enroulement de l'hélice ou d'ouverture de celle-ci.

Comme on l'a vu précédemment, un des avantages des compositions de l'invention est qu'elles fournissent un véhicule d'acide nucléique ne contenant pas de molécule à caractère cationique.

Toutefois, selon une variante de l'invention, il est possible d'inclure certains adjuvants cationiques, la présence des vésicules multilamellaires de l'invention permettant de masquer leur toxicité ou d'augmenter leur activité. Il semble que l'effet de certains adjuvants cationiques est de permettre de fixer de façon électrostatique les vésicules sur les parois des cellules, ce qui explique l'augmentation d'efficacité des vecteurs. De plus, il apparaît que le fait d'encapsuler une partie des molécules cationiques permet de diminuer leur cytotoxicité, l'encapsulation diminuant le pourcentage de molécules cationiques susceptibles d'entrer en contact avec les cellules environnantes.

L'encapsulation permet aussi de réduire la concentration en molécule cationique utilisée, grâce à l'effet de synergie qui en améliore l'efficacité.

Ainsi donc, il est possible de co-encapsuler dans les vésicules selon l'invention un adjuvant cationique en évitant les inconvénients connus selon l'art antérieur lors de l'utilisation de tels produits.

Les vésicules comprenant l'acide nucléique pourront également, selon des variantes particulièrement intéressantes de l'invention, être modifiées en surface en vue d'être mieux reconnues par la cible visée. Selon cette variante, on adjoindra aux vésicules des molécules, en général des protéines, qui leur permettront d'être reconnues spécifiquement par certaines cibles dans l'organisme, et donc d'augmenter d'une part la sélectivité de la transfection et d'autre part son efficacité. Plusieurs méthodes peuvent être utilisées, en particulier le recours aux anticorps monoclonaux. Dans tous les cas il est nécessaire de fixer le système de ciblage sur la surface de la vésicule, pour que son efficacité de reconnaissance soit maximale. Cette fixation peut être réalisée par des méthodes physiques (adsorption) ou bien chimiques. Dans les deux cas, la facilité d'obtention et de manipulation des vésicules de l'invention sont des atouts majeurs pour la réussite de ces greffages de fonctions à la surface des vecteurs. Une des applications importantes dans le domaine biomédical est l'utilisation de vésicules portant à leur surface des anticorps monoclonaux ou des fragments Fab, spécifiques de récepteurs de surface cellulaire impliqués, par exemple, dans la reconnaissance de particules virales (herpesvirus...) et agissant comme transducteur de la réponse cellulaire.

L'invention concerne, suivant d'autres caractéristiques essentielles, des compositions pharmaceutiques contenant des vésicules telles que définies précédemment renfermant différents acides nucléiques, en suspension dans un véhicule pharmaceutiquement acceptable.

Selon l'activité biologique ou pharmaceutique envisagée, différents types d'acides nucléiques peuvent être encapsulés.

Ainsi, l'encapsulation d'un gène complet pourra être réalisée en vue de fournir une composition pharmaceutique destinée à suppléer un gène déficitaire ou absent. A titre d'exemple, une telle utilisation pourra être faite dans le traitement de la mucoviscidose.

L'encapsulation de constructions nucléiques comme des oligo-nucléotides destinés à induire une surexpression ou une atténuation de l'activité d'un gène spécifique pourra être utilisée dans des applications en cancérologie ou en virologie.

On peut aussi utiliser ces vecteurs dans des modèles de vaccination à partir d'ADN codant pour une protéine antigénique.

On peut aussi vectoriser de l'ARN car la vectorisation d'une séquence nucléotidique complémentaire s'hybridant au transcrit inhibe la lecture de l'ARNm par les ribosomes et en empêche sa traduction en une protéine active.

L'invention concerne également l'utilisation des compositions définies précédemment comme agent destiné à transformer une lignée cellulaire.

Elle concerne donc un procédé de transformation *in vitro* d'une lignée cellulaire, consistant à traiter ladite lignée par une composition telle que décrite précédemment.

Il s'agit, dans ce cas plus précisément, d'amener un gène au niveau du noyau de la cellule de manière à ce qu'il soit intra-nucléaire et/ou intrachromosomique afin d'en modifier sa propre expression et/ou l'expression d'autres gènes codant pour une ou plusieurs protéines, soit d'immortaliser une lignée par incorporation d'un gène viral et/ou d'un oncogène viral.

Selon un avantage supplémentaire de la technologie de l'invention, elle fournit des vésicules permettant d'effectuer une transfection en présence de sérum, ce qui est impossible à réaliser avec des vésicules de l'art antérieur. Ceci constitue un progrès considérable qui permet d'envisager pour la première fois l'utilisation de vésicules à base de tensioactifs *in vivo.*

Ce point a été vérifié par des essais de transfection *in vitro* sur des lignées cellulaires, et sur des cultures primaires de cellules humaines différenciées. Des résultats de transfection aussi bons en présence qu'en absence de sérum de veau foetal ont pu être obtenus alors que les autres vecteurs artificiels connus à l'heure actuelle (lipides cationiques) ne fonctionnent pas dans un tel milieu. Ce dernier résultat est fondamental car il est une condition *sine qua non* de l'utilisation *in vivo.*

Les premiers résultats de transfection *in vivo* sont très encourageants. Ces résultats ont montré que le transfert de gènes pouvait se faire en site tumoral et en injection systémique. L'injection intraveineuse chez l'animal, de vésicules selon l'invention encapsulant le gène de la β-galactosidase permet d'induire une activité galactosidase dans différents organes (coeur, poumon, foie). Ce point est très encourageant pour l'utilisation à des fins thérapeutiques du procédé.

Ainsi, l'invention concerne également l'utilisation de vésicules multilamellaires à structure en oignon pour la préparation d'une composition pharmaceutique destinée à corriger ou pallier une fonction cellulaire déficiente ou altérée ou à apporter une nouvelle fonction à une cellule, notamment dans le domaine de la cancérologie, de la virologie ou de la correction des défauts osseux.

Ainsi, les applications de ce vecteur touchent plusieurs domaines de la recherche biomédicale, en particulier la cancérologie. Dans ce domaine, les premiers résultats ont été obtenus chez des souris portant des tumeurs. Des injections de vésicules encapsulant un gène rapporteur ont été effectuées et une activité génique a été identifiée au niveau de la tumeur. Il devient alors possible d'envisager l'utilisation de tels vecteurs portant des gènes codant pour des facteurs antimitotiques, ou impliqués dans les phénomènes d'apoptose (mort cellulaire) afin d'étudier l'effet de ce transfert de gènes sur la régression de la tumeur.

De plus, un autre avantage de ce vecteur est de fonctionner également *in vivo* au niveau d'un site d'implantation donné. Un autre domaine d'application de ces vecteurs concerne les méthodes actuelles de substitution osseuse. L'implantation dans un défaut osseux de vésicules portant le gène codant pour des protéines ostéoinductrices connues sous le sigle BMPs correspondant à la terminologie anglaise "Bone Morphogenetic Proteins" devrait favoriser l'ostéogénèse. Si l'avenir de ces facteurs ostéoinducteurs dans le domaine orthopédique est très prometteur, il reste néanmoins, à l'heure actuelle, à résoudre le problème majeur de la vectorisation, de la régulation du système de délivrance au site d'implantation sans observer d'effet systémique. La présente invention fournit pour la première fois un vecteur acceptable. Par ailleurs, les injections systémiques de vésicules portant le gène de la β-galactosidase, et les premières expériences de biodistribution ont montré une activité génique dans différents organes. L'utilisation d'un tel procédé associé à un ciblage, comme décrit précédemment, offre de grandes perspectives thérapeutiques.

Les exemples ci-dessous sont donnés en référence aux figures 1 à 3 qui représentent respectivement :
- figure 1 : l'histogramme des rayons exprimés en nm des vésicules multilamellaires préparées selon l'exemple 1 ;
- figure 2, donnée en référence à l'exemple 3, l'efficacité de la transfection du gène de la β-galactosidase sur des cellules fibroblastiques de peau humaine pour deux types de formulations différentes selon l'invention et un vecteur commercial ;
- figure 3, donnée en référence à l'exemple 4, les résultats comparatifs de l'efficacité de la transfection du gène de la β-galactosidase sur des fibroblastes humains en utilisant différentes formulations de microvésicules selon l'invention, contenant ou non un adjuvant cationique, en comparaison avec un vecteur commercial.

### EXEMPLES

Sauf indications contraires, les quantités et proportions données dans les exemples sont en poids.

### Exemple 1

### Protection vis-à-vis de la DNase

Cet exemple a pour but de montrer l'efficacité des microvésicules multilamellaires selon l'invention pour encapsuler un ADN et le protéger de l'action de l'enzyme DNase. Pour cela, un ADN greffé (ADN-DIG) est encapsulé selon le procédé de l'invention. Les microvésicules contenant l'ADN, ainsi que l'ADN libre, sont ensuite soumises à l'action de l'enzyme. L'intégrité de l'ADN est ensuité révélée par hybridation de l'ADN complémentaire et coloration. On observe alors que l'ADN encapsulé selon le procédé est intact, alors que l'ADN libre a été détruit par l'enzyme.

### a) Préparation de l'échantillon

10 µl d'une solution à 1 µg/50 µl d'ADN-DIG (Boehringer Mannheim), soit 200 ng d'ADN-DIG sont mélangés à 375 µl d'eau, 100 mg d'alcool laurique éthoxylé à 4 molécules d'oxyde d'éthylène (laureth 4 par exemple Lauropal-4-Witco) et 525 mg de lécithine de soja à 90 % de phosphatidylcholine (Phospholipon P90, Natterman). L'eau est préalablement stérilisée par filtration sur un filtre de 0,25 µm, tandis que les tensioactifs sont traités par rayonnement UV. Après mélange à température ambiante, au cours duquel on prend soin d'appliquer un cisaillement homogène et uniforme sur tout l'échantillon, on obtient une pâte, correspondant à la phase cristal-liquide, arrangée sous forme d'assemblée compacte de microvésicules. On laisse reposer cette pâte pendant 24 h.

Pour leur utilisation, on prépare une dispersion des microvésicules en diluant 50 mg de pâte dans 1 ml d'eau stérile.

### b) Caractérisation

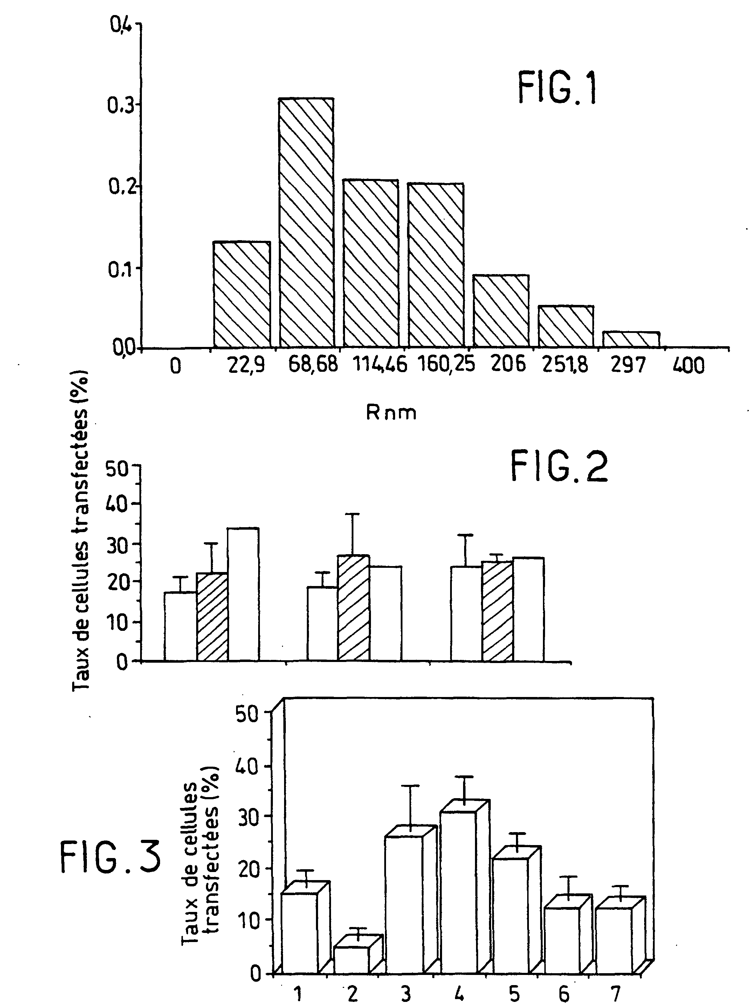

La taille des microvésicules est mesurée par diffusion dynamique de la lumière sur une dispersion à 1 % de la pâte dans l'eau. On obtient une valeur du diamètre de 0,2 µm environ. Cette valeur est confirmée par microscopie électronique (cryofracture). Les résultats de mesure de taille sont indiqués sur la figure 1 donnant l'histogramme de la distribution de taille, observée par microscopie. Un contrôle plus précis de la taille peut être obtenu en utilisant le procédé décrit dans le brevet WO-A-93 19735.

### c) Protocole d'essai

On prépare une dispersion de base des microvésicules contenant l'ADN à partir de 50 mg de pâte, dispersée dans 1 ml d'eau. Cette dispersion de base est ensuite diluée dans l'eau pour obtenir 5 dispersions d'essais, contenant de 10 ng/ml à 1 pg/ml d'ADN, par variation d'un facteur 10. On prépare des solutions d'ADN-DIG libre de mêmes concentrations pour servir de témoins.

Ces dispersions sont mises en contact pendant 1 h à 37°C avec une solution de DNase I (Boehringer Mannheim) dans une proportion de 2 unités de DNAse pour 1 µg d'ADN. Dans un deuxième temps, la présence ou non d'ADN-DIG est révélée par fixation sur une membrane de nitrocellulose (Hybond-C super) puis par hybridation avec l'ADN-DIG complémentaire en utilisant la technique du "dot-blot". La membrane est ensuite révélée par coloration aux NBT/BCIP suivant le protocole développé par Boehringer Mannheim et publié dans Genius, Applications Manuel, Boehringer-Mannheim Biochemicals Indianapolis 5-7, 1989.

### d) Résultats

On constate par analyse du "dot-blot" que :
. l'ADN libre non traité par la DNAse est visualisé par la technique du "dot-blot"
. l'ADN libre traité par la DNAse est totalement détruit, pour toutes les concentrations
. l'ADN encapsulé selon le procédé de l'invention, non traité à la DNAse est visualisé par cette même technique
. l'ADN encapsulé selon le procédé de l'invention, traité à la DNAse reste nettement visible, et est donc protégé de l'action enzymatique
. les microvésicules vides (sans ADN) ne donnent pas de réaction visible dans l'analyse, ce qui écarte tout risque de faux positif.

Une analyse plus fine de l'intensité comparée des images de "dot-blot" permet d'évaluer qu'environ 80 % de l'ADN a été protégé de l'action enzymatique. On peut en déduire que les 20 % détruits correspondent à l'ADN résiduel non encapsulé, ce qui permet d'évaluer le taux d'encapsulation aux environs de 80 %. Ces résultats ont été confirmés par une expérience d'électrophorèse sur un gel d'agarose, qui n'a montré aucune dégradation de l'ADN.

### Exemple 2

### Interaction entre l'ADN encapsulé et cellules en culture

Le but de cet exemple est de montrer qu'un ADN encapsulé selon le procédé de l'invention est capable de pénétrer à l'intérieur de cellules, d'y être relâché, et d'atteindre le noyau. Pour cela, on couple un ADN plasmidique à une sonde fluorescente, qui permettra une visualisation de l'incorporation cellulaire par microscopie de fluorescence.

### a) Préparation de l'échantillon

L'ADN plasmidique (pBR 322, de 4363 paires de base, Promega) à une concentration de 0,1 µg/ml est couplé à la fluorescéine (sonde YoYo-1, Molecular Probe Inc.) puis encapsulé dans des microvésicules multilamellaires, selon le même mode opératoire que pour l'exemple 1, avec :
solution aqueuse d'ADN YoYo : 37,5 %
Alcool laurique éthoxylé à 4 mole d'OE : 10 %
Lécithine de soja (90 % de phosphatidylcholine) : 52,5 %

### b) Incubation des cellules :

Les diverses lignées cellulaires, fibroblastes humains (culture primaire), NIH 3T3 (ATCC) sont maintenues dans des conditions classiques de culture (IMDM, Gibco, Life technology), contenant 10 % de sérum de veau (Gibco, Life technology), 10 000 U de pénicilline-streptomycine (Gibco, Life technology) à 37°C, en atmosphère à 5 % de CO₂. Les cellules sont mises en présence d'une dispersion à 10 % de microvésicules contenant l'ADN pendant 5 min, dans un milieu IMDM seul. Après ce temps d'incubation, les cellules sont lavées pour éliminer les dispersions de vésicules puis visualisées.

### c) Résultat

Les cellules sont visualisées par microscopie de fluorescence, après différentes durées d'incubation.
. A t = O, (pour contrôle, avant lavage) on observe les microvésicules dans le milieu surnageant comme des points fluorescents.
. A t = 5 min, les microvésicules fluorescentes sont visualisées au contact de la surface des cellules. On note le début d'une diffusion intracytoplasmique de la fluorescence.
. A t = 1 heure, on observe la diffusion intracytoplasmique de la fluorescence. Le pourtour nucléaire est nettement apparent, un début de fluorescence au niveau du noyau apparaît.
. Entre t = 2 et 8 heures, la fluorescence commence à apparaître dans le noyau. Elle reste forte dans le cytoplasme.
. A t = 48 heures, la fluorescence est plus faible et ne subsiste qu'à proximité des noyaux.

Cet exemple montre que l'ADN a pu être incorporé dans les fibroblastes, puis a traversé le cytoplasme pour atteindre le noyau.

### Exemple 3

### Encapsulation d'un gène et mise en évidence du transfert et de l'expression transitoire de ce gène

Le but de cet exemple est de montrer qu'un gène encapsulé dans les microvésicules multilamellaires de l'invention peut être incorporé dans le noyau d'une cellule et s'exprimer. Le test retenu est l'utilisation du gène qui code pour la β-galactosidase, et dont l'expression est mise en évidence par la coloration bleue des noyaux des cellules transfectées, lors de la réaction avec le substrat X-GAL. Dans cet exemple, l'efficacité de transfection est comparée à celle d'un vecteur commercial, le LipofectAce® (Life Technology).

### a) Préparation des microvésicules

Les microvésicules contenant le gène qui code pour la β-galactosidase (LacZ) sont préparées selon le mode opératoire de l'exemple 1, en utilisant une solution aqueuse du gène, à 10 mg/ml. Trois types de gène de type LacZ modifié ont été essayés : pRSCLacZ, pCHLacZ et pRSVLacZ-Sal-1. Chaque gène a été coencapsulé avec de la polylysine en concentration 100 µM, de poids moléculaire soit 3,5 kDa, soit 10 kDa. La composition des microvésicules est la suivante :

| | |
|---|---|
| Lécithine de soja à 90 % de phosphatidylcholine | 41,5 % |
| Cholestérol (Sigma) | 3,5 % |
| Oléate de potassium | 5 % |
| Solution aqueuse d'ADN et de polylysine | 49,5 %. |

Pour l'incubation des cellules, les microvésicules sont dispersées dans l'eau, afin d'obtenir un milieu d'incubation contenant 10 µg/ml d'ADN.

### b) Transfection

Les cellules (fibroblastes humains, culture primaire) sont maintenues dans des conditions classiques de culture comme dans l'exemple 2. Pour l'incubation, le milieu est remplacé par un milieu IMDM contenant 100 µM de chloroquine, et sans sérum. L'incubation avec les microvésicules dure de 2 à 12 heures. Les cellules sont ensuite lavées et maintenues en culture pendant 48 heures dans un milieu complet (IMDM, sérum, pénicilline-streptomycine).

La visualisation de la transfection est effectuée par lavage des cellules, fixation, et addition du substrat X-GAL (Biosynth AG). Ce substrat est clivé par l'enzyme correspondant au gène de la β-galactosidase, en donnant un précipité bleu foncé exclusivement intracellulaire dû au signal intranucléaire désigné généralement par "nls", selon la terminologie anglaise "nuclear localisation signal".

La même expérience est effectuée, pour comparaison, en utilisant un vecteur commercial LipofectAce® (Life Tcchnology), utilisé selon le protocole du fabricant, avec les mêmes concentrations de gène.

### c) Résultat

Les résultats, sous forme de pourcentage de cellules transfectées sont donnés sur la figure 2. Chaque histogramme correspond à un des gènes (de gauche à droite : pRSCLacZ, pCHLacZ et pRSVLacZ-Sal-1). Sur chacun, on a porté le pourcentage de transfection obtenu avec, de gauche à droite LipofectAce® , les microvésicules avec la polylysine de 3,5 kDa et les microvésicules avec la polylysine de 10 kDa.

On constate que, dans tous les cas, les résultats sont meilleurs en utilisant les microvésicules qu'avec le vecteur commercial. Des pourcentages de 25 à 35 % de transfection sont obtenus dans le cas des microvésicules.

Un test à la DNAse identique à celui décrit dans l'exemple 1 peut être effectué sur ce gène rapporteur, en utilisant pour la technique du "dot-blot" un cDNA marqué au ³²P. On constate alors que, comme dans l'exemple 1, l'ADN encapsulé dans les microvésicules selon l'invention n'est pas détruit par la DNAse. Par contre, le rendement d'encapsulation observé est plus faible. Le même test de protection vis-à-vis de la DNAse effectué sur ce même gène vectorisé par le produit commercial ne montre aucune protection de ce type de vecteur, ce qui pourrait expliquer le faible taux de transfection obtenu pour ce produit commercial.

### Exemple 4

### Encapsulation d'un gène et mise en évidence du transfert et de l'expression transitoire de ce gène : effet d'adjuvants cationiques

Cet exemple a pour but de mettre en évidence la possibilité d'utilisation d'adjuvants de type cationique pour améliorer l'efficacité de la transfection. L'adjuvant utilisé est un polymère, la polyéthylèneimine. Plusieurs essais sont réalisés, dans des conditions analogues à celles de l'exemple 3 (transfection du gène de la β-galactosidase), mais sans utiliser de polylysine. Pour comparaison deux formulations avec polylysine, mais sans adjuvant, sont préparées, l'une identique à celle de l'exemple 3 (lécithine, oléate de potassium, cholestérol), l'autre identique à celle de l'exemple 1 (lécithine, laureth 4). Enfin, pour contrôle, trois expériences de transfection sont effectuées en utilisant soit un vecteur commercial LipofectAce® (Life Technology), soit de l'ADN non encapsulé, complexé à la polyéthylèneimine.

### a) Préparation des microvésicules

Le mode opératoire est strictement identique à celui utilisé dans l'exemple 3, en utilisant comme gène le pRSVLacZ. La polyéthylèneimine (masse molaire 50 kDa, Sigma) est additionnée à la solution aqueuse d'ADN avant encapsulation, à deux concentrations : 10 mM et 100 µM.

Les témoins sans polyéthylèneimine sont préparés selon le mode opératoire de l'exemple 3 (lécithine, cholestérol, oléate de potassium), ou de l'exemple 1 (lécithine, laureth 4).

### b) Transfection

Le protocole est identique à celui de l'exemple 3. La transfection est effectuée sur des fibroblastes humains (culture primaire). Dans tous les cas, la concentration en gène dans le milieu d'incubation est de 10 µg/ml.

Les expériences avec l'ADN non encapsulé sont effectuées en introduisant dans le milieu d'incubation, à la place de la dispersion de vésicules selon l'invention, une solution contenant l'ADN et la polyéthylèneimine préalablement mélangés.

### c) Résultat

Les résultats, donnés sous la forme d'un histogramme des pourcentages de cellules transfectées, sont donnés sur la figure 3. Les résultats correspondent, de gauche à droite aux essais suivants :
1-microvésicules à base de lécithine et d'oléate de potassium
2-microvésicules à base de lécithine et d'alcool laurique à 4 molécule d'oxyde d'éthylène (laureth 4)
3-microvésicules à base de lécithine et d'oléate de potassium, co-encapsulant la polyéthylèneimine, en concentration 10 mM
4-microvésicules à base de lécithine et d'oléate de potassium, co-encapsulant la polyéthylèneimine, en concentration 100 µM
5-ADN complexé au poléthylèneimine, avec une concentration en polymère de 10 mM
6-ADN complexé à la polyéthylèneimine, avec une concentration en polymère de 100 µM
7-utilisation du vecteur commercial LipofectAce® (Life Technology).

On observe que grâce à la co-encapsulation de la polyéthylèneimine, le taux de cellules transfectées atteint 35 % . L'ADN non encapsulé, simplement complexé par la polyéthylèneimine est aussi transfecté, mais présente alors un moins bon taux de transfection. Tous les essais effectués avec des vecteurs basés sur l'oléate de potassium, avec ou sans adjuvant, donnent un résultat meilleur que le vecteur commercial.

### Exemple 5

### Encapsulation d'un gène et mise en évidence du transfert et de l'expression transitoire de ce gène : effet d'adjuvants non cationiques

Cet exemple a pour but de mettre en évidence la possibilité de co-encapsulation d'adjuvants non cationiques de condensation de l'ADN pour améliorer l'efficacité de transfection. L'adjuvant utilisé est un mélange d'histones H1, H2a, H2b, H3, H4 de thymus de veau (fournisseur : Boehringer). L'ADN utilisé est le même que celui utilisé dans l'exemple 4.

### a) Condensation de l'ADN par les histones

L'ADN est préalablement condensé par mise en solution simultanée d'un poids égal d'ADN et du mélange d'histones H1, H2a, H2b, H3, H4 de thymus de veau (50µg d'ADN avec 50 µg de mélange d'histones dans 12 µl d'eau) et incubation du mélange pendant 10 min à 37°C.

### b) Préparation des microvésicules

Le mode opératoire est strictement identique à celui utilisé dans l'exemple 3, en utilisant à la place de la solution de gène, la solution de mélange ADN/histones.

La composition en poids des vésicules est :

| | |
|---|---|
| lécithine de soja à 90% de phosphatidyl choline : | 31,5 % |
| cholesterol | 6,5 % |
| alcool laurique éthoxylé à 4 OE | 2 % |
| solution aqueuse du mélange ADN/histones | 60 % |

### c) Mesure du rendement d'encapsulation

1µg d'ADN a été marqué au ³²P (selon la méthode dite du random priming) et mélangé avec 49 µg d'ADN non marqué (pour respecter la quantité de 50 µg d'ADN). Cet ADN est encapsulé puis les vésicules sont dispersées dans de l'eau. La suspension est ultracentrifugée à 30 000 rpm pendant 45 minutes. Le surnageant est séparé du culot et chacun est mis à compter dans un compteur β pour évaluer la radioactivité présente.

Le rendement d'encapsulation mesuré est toujours supérieur à 80%

### d) Transfection

Les essais de transfection sont effectués sur des fibroblastes de peau humaine selon un protocole d'incubation et de comptage identique à celui décrit dans les exemples 3. La concentration d'ADN utilisée dans la dispersion mise au contact des cellules est de 5µg/ml.

Le pourcentage de transfection dans ces conditions varie de 20 à 30 % (défini comme le nombre de cellules transfectées par rapport aux cellules incubées).

## Revendications

1. Composition contenant au moins un acide nucléique dont au moins une partie se trouve incluse à l'intérieur de vésicules multilamellaires, **caractérisée en ce que** lesdites vésicules sont constituées de leur centre jusqu'à leur périphérie de bicouches comprenant au moins un agent tensioactif, concentriques et séparées par un milieu liquide.

2. Composition selon la revendication 1, **caractérisée en ce que** lesdites vésicules ont des dimensions inférieures à 1 µm, de préférence comprises entre 0,1 et 1 µm.

3. Composition selon l'une des revendications 1 ou 2, **caractérisée en ce que** les tensioactifs constituant lesdites bicouches desdites vésicules sont des tensioactifs non cationiques.

4. Composition selon la revendication 3, **caractérisée en ce que** lesdits agents tensioactifs non cationiques sont choisis dans le groupe constitué :
- des phospholipides hydrogénés ou non hydrogénés,
- des acides gras en C₆ à C₁₈, saturés ou mono- ou polyinsaturés, linéaires ou ramifiés, sous forme d'acide ou de sel d'un métal alcalin, alcalino-terreux ou d'une amine,
- des esters, éthoxylés ou non, de ces mêmes acides gras et
. de saccharose,
. de sorbitan,
. de mannitol,
. de glycérol ou de polyglycérol,
. de glycol,
- des mono-, di- ou triglycérides ou des mélanges de glycérides de ces mêmes acides gras,
- des alcools gras en C₆ à C₁₈, saturés ou mono- ou polyinsaturés, linéaires ou ramifiés, éthoxylés ou non,
- des éthers, éthoxylés ou non, de ces mêmes alcools gras et
. de saccharose,
. de sorbitan,
. de mannitol,
. de glycérol ou de polyglycérol,
. de glycol.
- des huiles végétales polyéthoxylées, hydrogénées ou non hydrogénées,
- des polymères séquencés de polyoxyéthylène et de polyoxypropylène (poloxamères),
- de l'hydroxystéarate de polyéthylèneglycol,
- des alcools à squelette stérol tel que le cholestérol, le sistostérol.

5. Composition selon l'une des revendications 1 à 4, **caractérisée en ce que** les bicouches desdites vésicules comprenant au moins deux agents tensioactifs dont l'un présente une balance lipophile hydrophile (HLB) comprise entre 1 et 6 et l'autre une balance lipophile hydrophile (HLB) comprise entre 3 et 15.

6. Composition selon l'une des revendications 1 à 5, **caractérisée en ce qu'**au moins 10 %, et de préférence au moins 40 % dudit(desdits) acide(s) nucléique(s), se trouvent inclus à l'intérieur desdites vésicules multilamellaires.

7. Composition selon l'une des revendications 1 à 6, **caractérisée en ce que** 60 à 100 % dudit ou desdits acides nucléiques se trouvent inclus dans lesdites vésicules.

8. Composition selon l'une des revendications 1 à 7, **caractérisée en ce que** ledit acide nucléique est de l'ADN ou une séquence nucléotidique d'ADN.

9. Composition selon l'une des revendications 1 à 7, **caractérisée en ce que** ledit acide nucléique est un gène particulier ou une séquence dudit gène, en particulier une séquence codant pour une protéine donnée.

10. Composition selon l'une des revendications 1 à 7, **caractérisée en ce que** ledit acide nucléique est de l'ARN ou une séquence nucléotidique d'ARN.

11. Composition selon l'une des revendications 1 à 7, **caractérisée en ce que** ledit acide nucléique est un oligonucléotide sens et/ou anti-sens.

12. Composition selon l'une des revendications 1 à 11, **caractérisée en ce que** lesdites vésicules contiennent, en outré, un adjuvant cationique, en particulier une poléthylèneimine, coencapsulé avec ledit acide nucléique.

13. Composition selon l'une des revendications 1 à 12, **caractérisée en ce qu'**elle contient au moins un produit co-encapsulé choisi dans le groupe des agents de condensation de l'ADN, en particulier parmi les histones, des enzymes d'intégration ou de recombinaison, des enzymes destinées à optimiser la réplication de l'acide nucléique encapsulé, telles que les topoisomérases ou les hélicases.

14. Composition selon l'une des revendications 1 à 13, **caractérisée en ce que** lesdites vésicules sont modifiées en surface par l'adjonction de molécules leur permettant d'être reconnues spécifiquement par une cible dans l'organisme, en particulier par adjonction d'anticorps monoclonaux ou de fragments Fab.

15. Procédé de préparation d'une composition selon l'une des revendications 1 à 14, **caractérisé en ce qu'**il comprend la préparation d'une phase cristal-liquide lamellaire incorporant ledit acide nucléique et le réarrangement de ladite phase cristal-liquide en vésicules multilamellaires par application d'un cisaillement.

16. Composition pharmaceutique contenant des vésicules telles que définis dans l'une des revendications 1 à 14, en suspension dans un véhicule pharmaceutiquement acceptable.

17. Composition pharmaceutique selon la revendication 16, **caractérisée en ce que** ledit acide nucléique encapsulé est un gène complet, en particulier un gène destiné à suppléer un gène déficitaire ou absent.

18. Composition pharmaceutique selon la revendication 16, **caractérisée en ce que** ledit acide nucléique est un oligonucléotide destiné à induire une surexpression ou une atténuation de l'activité d'un gène spécifique.

19. Composition pharmaceutique selon la revendication 16, **caractérisée en ce qu'**il s'agit d'un vaccin, ledit acide nucléique étant un ADN codant pour une protéine antigénique.

20. Utilisation de vésicules multilamellaires à structure en oignon telles qu'elles sont définies dans l'une des revendications 1 à 14 pour la préparation d'une composition pharmaceutique destinée à corriger ou pallier une fonction cellulaire déficiente ou altérée ou à apporter une nouvelle fonction à une cellule, notamment dans le domaine de la cancérologie, de la virologie ou de la correction des défauts osseux.

21. Procédé de transformation *in vitro* d'une lignée cellulaire, **caractérisé en ce qu'**il consiste à traiter ladite lignée par une composition selon l'une des revendications 1 à 14.

## Claims

1. A composition containing at least one nucleic acid at least one part of which is found included inside multilamellar vesicles, **characterised in that** said vesicles are constituted from their centers to their peripheries of bi-layers comprising at least one surfactant agent, said bi-layers being concentric and separated by a liquid medium.

2. The composition according to claim 1, **characterised in that** said vesicles have dimensions of less than 1 µm, preferably between 0.1 and 1 µm.

3. The composition according to one of claims 1 or 2, **characterised in that** the surfactants constituting said bi-layers of said vesicles are non-cationic surfactants.

4. The composition according to claim 3, **characterised in that** said non-cationic surfactant agents are selected from the group consisting of:
- hydrogenated or non-hydrogenated phospholipids,
- saturated or mono- or polyunsaturated, linear or branched C₆ to C₁₈ fatty acids, in the form of an acid or an alkali metal salt, an alkaline earth metal salt, or an amine salt,
- ethoxylated or non-ethoxylated esters of the same fatty acids and
. of sucrose,
. of sorbitan,
. of mannitol,
. of glycerol or of polyglycerol,
. of glycol,
- mono-, di- or triglycerides or mixtures of glycerides of the same fatty acids,
- saturated or mono- or polyunsaturated, linear or branched, ethoxylated or non-ethoxylated C₆ to C₁₈ fatty alcohols,
- ethoxylated or non-ethoxylated ethers of the same fatty alcohols and
. of sucrose,
. of sorbitan,
. of mannitol,
. of glycerol or of polyglycerol,
. of glycol,
- hydrogenated or non hydrogenated polyethoxylated vegetable oils,
- block polymers of polyoxyethylene and polyoxypropylene (poloxamers),
- polyethylene glycol hydroxystearate,
- sterol-skeleton alcohols such as cholesterol, or sistosterol.

5. The composition according to one of claims 1 to 4, **characterised in that** the bi-layers of said vesicles comprising at least two surfactant agents one of which has a hydrophilic lipophilic balance (HLB) between 1 and 6 and the other a hydrophilic lipophilic balance (HLB) between 3 and 15.

6. The composition according to one of claims 1 to 5, **characterised in that** at least 10 %, and preferably at least 40 %, of said nucleic acid(s) are found included inside said multilamellar vesicles.

7. The composition according to one of claims 1 to 6, **characterised in that** 60 to 100 % of said nucleic acid(s) are found included in said vesicles.

8. The composition according to one of claims 1 to 7, **characterised in that** said nucleic acid is DNA or a nucleotide sequence of DNA.

9. The composition according to one of claims 1 to 7, **characterised in that** said nucleic acid is a particular gene or a sequence of said gene, particularly a sequence encoding a given protein.

10. The composition according to one of claims 1 to 7, **characterised in that** said nucleic acid is RNA or a nucleotide sequence of RNA.

11. The composition according to one of claims 1 to 7, **characterised in that** said nucleic acid is a sense and/or antisense oligonucleotide.

12. The composition according to one of claims 1 to 11, **characterised in that** said vesicles further contain a cationic adjuvant, particularly a polyethyleneimine, co-encapsulated with said nucleic acid.

13. The composition according to one of claims 1 to 12, **characterised in that** it contains at least one co-encapsulated product selected from the group of DNA condensing agents, particularly from the histones, integration or recombination enzymes, enzymes intended to optimise the replication of the encapsulated nucleic acid, such as the topoisomerases or the helicases.

14. The composition according to one of claims 1 to 13, **characterised in that** said vesicles are modified on the surface by the addition of molecules enabling them to be recognised specifically by a target in the organism, particularly by the addition of monoclonal antibodies or of Fab fragments.

15. A method of preparation of a composition according to one of claims 1 to 14, **characterised in that** it comprises the preparation of a lamellar liquid crystal phase incorporating said nucleic acid and the rearrangement of said liquid crystal phase into multilamellar vesicles by the application of a shearing.

16. A pharmaceutical composition containing vesicles as defined in one of claims 1 to 14, in suspension in a pharmaceutically acceptable vehicle.

17. The pharmaceutical composition according to claim 16, **characterised in that** said encapsulated nucleic acid is a complete gene, particularly a gene intended to compensate for an absent or a deficient gene.

18. The pharmaceutical composition according to claim 16, **characterised in that** said nucleic acid is an oligonucleotide intended to induce an over-expression of or a reduction in the activity of a specific gene.

19. The pharmaceutical composition according to claim 16, **characterised in that** it is a vaccine, said nucleic acid being a DNA encoding an antigenic protein.

20. Use of multilamellar vesicles of onion structure as defined in one of claims 1 to 14 for the preparation of a pharmaceutical composition intended to correct or to alleviate a deficient or altered cell function or to bring about a new function to a cell, notably in the field of cancerology, of virology or in the correction of bone defects.

21. A method of *in vitro* transformation of a cell line, **characterised in that** it consists in treating said line with a composition according to one of claims 1 to 14.

## Patentansprüche

1. Zusammensetzung, die mindestens eine Nucleinsäure enthält, die mindestens teilweise im Innern von multilamellaren Vesicula eingeschlossen ist, **dadurch gekennzeichnet, dass** die genannten Vesicula ab ihrem Zentrum bis zu ihrer Peripherie aus konzentrischen und durch ein flüssiges Medium voneinander getrennten Doppelschichten bestehen, die mindestens ein Tensid umfassen.

2. Zusammensetzung nach Anspruch 1, **dadurch gekennzeichnet, dass** die genannten Vesicula Dimensionen von weniger als 1 µm, vorzugsweise zwischen 0,1 und 1 µm, haben.

3. Zusammensetzung nach einem der Ansprüche 1 oder 2, **dadurch gekennzeichnet, dass** es sich bei den Tensiden, welche die genannten Doppelschichten der genannten Vesicula aufbauen, um nicht-kationische Tenside handelt.

4. Zusammensetzung nach Anspruch 3, **dadurch gekennzeichnet, dass** die genannten nicht-kationischen Tenside ausgewählt sind aus der Gruppe, die besteht aus:
- hydrierten oder nicht-hydrierten Phospholipiden,
- linearen oder verzweigten, gesättigten oder einfach oder mehrfach ungesättigten C₆-C₁₈-Fettsäuren in Form der Säure oder in Form eines Alkalimetall-, Erdalkalimetall- oder Aminsalzes,
- ethoxylierte oder nicht ethoxylierte Ester der genannten Fettsäuren mit
. Saccharose,
. Sorbitan,
. Mannit,
. Glycerin oder Polyglycerin,
. Glycol,
- Mono-, Di- oder Triglyceriden oder Mischungen von Glyceriden der genannten Fettsäuren,
- linearen oder verzweigten, ethoxylierten oder nicht ethoxylierten, gesättigten oder einfach oder mehrfach ungesättigten C₆-C₁₈-Fettalkoholen,
- ethoxylierten oder nicht ethoxylierten Ethem der genannten Fettalkohole mit
. Saccharose,
. Sorbitan,
. Mannit,
. Glycerin oder Polyglycerin,
. Glycol,
- hydrierten oder nicht hydrierten polyethoxylierten Pflanzenölen,
- Sequenz-Polymeren von Polyoxyethylen und Polyoxypropylen (Poloxameren),
- Polyethylenglycol-hydroxystearat,
- Alkoholen mit einem Sterin-Gerüst wie Cholesterin, Sitosterin.

5. Zusammensetzung nach einem der Ansprüche 1 bis 4, **dadurch gekennzeichnet, dass** die Doppelschichten der genannten Vesicula mindestens zwei Tenside umfassen, von denen eines ein hydrophiles-lipophiles Gleichgewicht (HLB) zwischen 1 und 6 und das andere ein hydrophiles-lipophiles Gleichgewicht (HLB) zwischen 3 und 15 aufweisen.

6. Zusammensetzung nach einem der Ansprüche 1 bis 5, **dadurch gekennzeichnet, dass** mindestens 10 %, vorzugsweise mindestens 40 %, der genannten Nucleinsäure(n) im Innern der genannten multilamellaren Vesicula eingeschlossen sind.

7. Zusammensetzung nach einem der Ansprüche 1 bis 6, **dadurch gekennzeichnet, dass** 60 bis 100 % der genannten Nucleinsäure(n) im Innern der genannten Vesicula eingeschlossen sind.

8. Zusammensetzung nach einem der Ansprüche 1 bis 7, **dadurch gekennzeichnet, dass** es sich bei der genannten Nucleinsäure um die DNA oder eine Nucleotid-Sequenz der DNA handelt.

9. Zusammensetzung nach einem der Ansprüche 1 bis 7, **dadurch gekennzeichnet, dass** es sich bei der genannten Nucleinsäure um ein spezielles Gen oder eine Sequenz des genannten Gens, insbesondere um eine für ein gegebenes Protein codierende Sequenz handelt.

10. Zusammensetzung nach einem der Ansprüche 1 bis 7, **dadurch gekennzeichnet, dass** es sich bei der genannten Nucleinsäure um die RNA oder eine Nucleotid-Sequenz der RNA handelt.

11. Zusammensetzung nach einem der Ansprüche 1 bis 7, **dadurch gekennzeichnet, dass** es sich bei der genannten Nucleinsäure um ein codierendes und/oder nicht-codierendes Oligonucleotid handelt.

12. Zusammensetzung nach einem der Ansprüche 1 bis 11, **dadurch gekennzeichnet, dass** die genannten Vesicula außerdem ein kationisches Adjuvans, insbesondere ein Polyethylenimin, enthalten, das zusammen mit der genannten Nucleinsäure co-eingekapselt ist.

13. Zusammensetzung nach einem der Ansprüche 1 bis 12, **dadurch gekennzeichnet, dass** sie mindestens ein coeingekapseltes Produkt enthält, das ausgewählt ist aus der Gruppe der DNA-Kondensationsagentien, insbesondere unter den Histonen, der Integrations- oder Rekombinations-Enzyme, der für die Optimierung der Replikation der eingekapselten Nucleinsäure bestimmten Enzyme wie der Topoisomerasen oder Helicasen.

14. Zusammensetzung nach einem der Ansprüche 1 bis 13, **dadurch gekennzeichnet, dass** die genannten Vesicula an der Oberfläche modifiziert sind durch Zuordnung (Addition) von Molekülen, die es ihnen erlauben, von einem Target in dem Organismus spezifisch erkannt zu werden, insbesondere durch Zuordnung (Addition) von monoklonalen Antikörpern oder Fab-Fragmenten.

15. Verfahren zur Herstellung einer Zusammensetzung nach einem der Ansprüche 1 bis 14, **dadurch gekennzeichnet, dass** es umfasst die Herstellung einer Kristall-Flüssigkeits-Lamellenphase, welche die genannte Nucleinsäure enthält, und die Umlagerung der genannten Kristall-Flüssigkeits-Phase zu multilamellaren Vesicula durch Anwendung einer Scherkraft.

16. Pharmazeutische Zusammensetzung, die Vesicula, wie sie in einem der Ansprüche 1 bis 14 definiert sind, suspendiert in einem pharmazeutisch akzeptablen Vehiculum enthält.

17. Pharmazeutische Zusammensetzung nach Anspruch 16, **dadurch gekennzeichnet, dass** es sich bei der genannten eingekapselten Nucleinsäure um ein vollständiges Gen, insbesondere ein Gen handelt, das ein defektes Gen oder ein fehlendes Gen ersetzen soll.

18. Pharmazeutische Zusammensetzung nach Anspruch 16, **dadurch gekennzeichnet, dass** es sich bei der genannten Nucleinsäure um ein Oligonucleotid handelt, das dazu bestimmt ist, eine Überexpression oder eine Schwächung der Aktivität eines spezifischen Gens zu induzieren.

19. Pharmazeutische Zusammensetzung nach Anspruch 16, **dadurch gekennzeichnet, dass** es sich um einen Impfstoff handelt, wobei die genannte Nucleinsäure eine DNA ist, die für ein Antigen-Protein codiert.

20. Verwendung von multilamellaren Vesicula mit Zwiebel-Struktur, wie sie in einem der Ansprüche 1 bis 14 definiert sind, zur Herstellung einer pharmazeutischen Zusammensetzung zum Korrigieren oder Beseitigen einer defekten oder veränderten Zellfunktion oder zur Einführung einer neuen Funktion in eine Zelle, insbesondere auf dem Gebiet der Kanzerologie, der Virologie oder der Korrektur von Knochendefekten.

21. Verfahren zur in vitro-Transformation einer Zellinie, **dadurch gekennzeichnet, dass** es darin besteht, dass man die genannte Zellinie mit einer Zusammensetzung nach einem der Ansprüche 1 bis 14 behandelt.
